# EUROPÄISCHE PATENTANMELDUNG

(11) **EP 0 830 844 A1**
(43) Veröffentlichungstag der Anmeldung: **25.03.1998**
(21) Anmeldenummer: 97120475.5
(22) Anmeldetag: 12.11.1993
(51) Int. Cl.: A61B 17/17, A61B 17/92, B25C 3/00

(54) **Setzwerkzeug für Befestigungsnagel**

(30) Priorität: 04.01.1993 DE 4300039
(62) Teilanmeldung aus: 93118401.4
(71) Anmelder: Kirsch, Axel, Dr., 70794 Filderstadt (DE); ALTATEC Medizintechnische Elemente GmbH & Co. KG., 75449 Wurmberg (DE)
(72) Erfinder: Kirsch, Axel, Dr., 70794 Filderstadt (DE); Dürr, Walter, 75196 Remchingen (DE)
(74) Vertreter: Goddar, Heinz J., Dr.

(57) **Zusammenfassung**

Setzwerkzeug zum Einsetzen eines Befestigungsnagels umfassend einen Nagelkopf und einen daran anschließenden Nagelschaft, welches ein langgestrecktes bleistiftartiges Griffteil, an dessem einen Ende ein Druck-Schlag-Kopf und an dessen anderem Ende eine mit mindestens zwei federnden Greifbacken versehene Haltevorrichtung für einen Befestigungsnagel vorgesehen ist, umfaßt.

## Beschreibung

Die Erfindung betrifft ein Setzwerkzeug zum Einsetzen eines Befestigungsnagels.

Es ist üblich, beispielsweise im Bereich der Kieferchirugie, Defektstellen in einem körpereigenen Knochen mit einem Knochenersatz- oder Aufbaumaterial auszufüllen, welches beispielsweise aus Hydroxylapatitgranulat, ggf. vermischt mit körpereigenem Knochengranulat, besteht. Die mit dem Knochenaufbaumaterial gefüllte Defektstelle wird dann mit einer Abdeckmembran aus körperfreundlichem Kunststoffmaterial oder dergleichen abgedeckt, welche benachbart zu der Defektstelle am körpereigener Knochen anliegt. Dies ist wichtig, damit das Knochenaufbaumaterial vom körpereigenen Knochen her knöchern durchwachsen wird, während es unerwünscht ist, daß andersartiges Bindegewebe, insbesondere Schleimhautgewebe, das Knochenersatzmaterial durchwächst, da es in diesem Fall nicht zu einem befriedigenden Ausfüllen der Knochendefektstelle mit nachwachsendem Knochenmaterial kommt.

Bislang ist es schwierig, die Abdeckmembran zuverlässig und dicht um die Defektstelle herum am körpereigenen Knochen festzulegen, so daß ein unerwünschtes Einwachsen von Nicht-Knochengewebe in die Knochendefektstelle nicht zuverlässig genug vermieden werden kann.

Der Erfindung liegt die Aufgabe zugrunde, einen Befestigungsnagel nebst Setzwerkzeug zu dessen Einsetzen zu schaffen, mittels dessen es möglich ist, die Abdeckmembran so zuverlässig und dicht auf einfache Weise mit dem körpereigenen bzw. -festen Knochen zu verbinden, daß ein ausschließlich oder zumindest nahezu weitgehendes Durchwachsen des Knochenaufbaumaterials mit Knochengewebe gewährleistet werden kann.

Erfindungsgemäß wird diese Aufgabe in Weiterbildung des gattungsgemäßen Befestigungsnagels dadurch gelöst, daß der Übergangsbereich zwischen dem Halte- und dem Spitzenteil eine Ringschulter vergrößerten Durchmessers aufweist, die zur Nagelspitze hin mit einer Konusfläche schräg in die Mantelfläche des Spitzenteiles übergeht und zum Nagelkopf hin widerhakenartig mit einer im wesentlichen senkrecht zur Längsachse des Befestigungsnagels verlaufenden Ringfläche an das Halteteil anschließt.

Dabei kann vorgesehen sein, daß die Konusfläche der Ringschulter vom Außenumfang der Ringfläche ohne wesentliche Konizitätsanderung in die Mantelfläche des Spitzenteils übergeht.

Die Erfindung sieht auch vor, daß die Konusfläche der Ringschulter mit der Längsachse des Befestigungsnagels einen größeren Winkel einschließt als der hieran anschließende Bereich der Mantelfläche des Spitzenteiles.

Eine weitere Ausführungsform des Befestigungsnagels nach der Erfindung ist dadurch gekennzeichnet, daß der Winkel zwischen der Konusfläche der Ringschulter und der Längsachse des Befestigungsnagels ca. 25° beträgt.

Der Befestigungsnagel kann weiterhin auch so ausgebildet sein, daß die Mantelfläche des Halteteiles mit einer Anschrägung in die im wesentlichen senkrecht zur Längsachse des Befestigungsnagels verlaufende Preßfläche des Nagelkopfes übergeht.

Bei der Erfindung kann auch vorgesehen sein, daß der Nagelkopf an seinem Umfangsrand im Anschluß an die Preßfläche eine von dieser in Richtung auf die dem Nagelschaft abgewandte Kopffläche des Nagelkopfes ansteigende Umfangsschrägung aufweist.

Ferner sieht die Erfindung bei dem vorgeschlagenen Befestigungsnagel vor, daß die Kopffläche des Nagelkopfes abgerundet ausgebildet ist.

Auch kann vorgesehen sein, daß als Herstellungsmaterial eine Titanlegierung oder dergleichen verwendet ist.

Das Setzwerkzeug nach der Erfindung zum Einsetzen eines Befestigungsnagels umfassend einen Nagelkopf und einen daran anschließenden Nagelschaft ist gekennzeichnet durch ein langgestrecktes bleistiftartiges Griffteil, an dessen einem Ende ein Druck-Schlag-Kopf und an dessen anderem Ende eine mit mindestens zwei federnden Greifbacken versehene Halteeinrichtung für einen Befestigungsnagel vorgesehen ist.

Dabei kann vorgesehen sein, daß die Halteeinrichtung vier durch eine Kreuzschlitzanordnung getrennte Greifbacken aufweist:

Ferner sieht die Erfindung hierbei vor, daß die Greifbacken einstückig mit dem daran anschließenden Bereich des Mantels des Griffteiles ausgebildet sind.

Auch schlägt die Erfindung vor, daß die Greifbacken jeweils eine Anlageschulter für die Kopffläche des Befestigungsnagels aufweisen, deren Abstand von dem dem Griffteil abgewandten Stirnende der Halteeinrichtung kleiner ist als die Höhe des Nagelkopfes.

Eine weitere Ausbildungsform des Setzwerkzeuges nach der Erfindung ist gekennzeichnet durch eine von dem Griffteil unabhängige Setzhilfe, mit einem Griffteil, einem daran anschließenden Fixierteil, welches einen mit einer Setzöffnung versehenen, an den Knochen in Anlage bringbaren Haltefuß aufweist, deren Durchmesser geringfügig größer ist als der Außendurchmesser der Halteeinrichtung, und einer in dem Fixierteil längsverschiebbaren Bohrschablone aus biegsamem Blech oder dergleichen, die an ihrem der Setzöffnung benachbarten Ende eine Bohröffnung aufweist und aus einer Bohrstellung, in der die Setzöffnung bis auf die Bohröffnung verschlossen ist, in eine Setzstellung zurückziehbar ist, in der die Setzöffnung freiliegt.

Dabei kann vorgesehen sein, daß der Haltefuß an der an dem Knochen ansetzbaren Unterseite mindestens zwei spitze Haltedorne aufweist.

Die Erfindung schlägt auch vor, daß diametral gegenüberliegend zu beiden Seiten der Setzöffnung je ein Haltedorn vorgesehen ist.

Eine weitere Ausführungsform der Erfindung sieht vor, daß die Bohrschablone nahe dem Griffteil mit einem von einem Operateur ohne Loslassen des Griffteiles betätigbaren Betätigungselement versehen ist.

Der Erfindung liegt die überraschende Erkenntnis zugrunde, daß es gelingt, die bisher bestehenden Probleme beim Abdecken von mit Knochenaufbaumaterial gefüllten Defektstellen dadurch zu lösen, daß die Abdeckmembran mittels eines Befestigungsnagels zuverlässig in der Umgebung der Defektstelle an dem körperfesten Knochen festgelegt wird, welcher infolge seiner widerhakenartigen Ausbildung ein dichtes Anpressen der Abdeckmembran an den körpereigenen Knochen gewährleistet. Wird die bevorzugte Ausführungsform des erfindungsgemäßen Setzwerkzeuges verwendet, bei der eine Setzhilfe Verwendung findet, so läßt sich mittels der Setzhilfe zunächst die Abdeckmembran unter seitlicher Festlegung durch die Haltedornen des Haltefußes der Setzhilfe exakt und fest an dem Knochen fixieren. Nachdem eine Setzbohrung eingebracht worden ist, die exakt in der Mitte der Setzöffnung liegt, läßt sich nach Zurückziehen der Bohrschablone durch die Setzöffnung hindurch mittels des Setzwerkzeuges ein Befestigungsnagel genau und ohne seitliches Verrücken in die Setzbohrung einsetzen und tief und fest in den Knochen einbringen, woraufhin die Abdeckmembran zuverlässig und dicht am Knochen fixiert ist.

Weitere Merkmale und Vorteile der Erfindung ergeben sich aus der nachstehenden Beschreibung, in der Ausführungsbeispiele anhand der schematischen Zeichnung im einzelnen erläutert sind. Dabei zeigt:
- Fig. 1: ein Ausführungsbeispiel eines Setzwerkzeuges nach der Erfindung im Schnitt durch die Längsmittelachse, in Verbindung mit, in Explosionsstellung gezeigt, einem Ausführungsbeispiel eines Befestigungsnagels nach der Erfindung;
- Fig. 2: eine Halteeinrichtung des Setzwerkzeuges von Figur 1 im axialen Längsschnitt, mit darin gehaltenem Befestigungsnagel;
- Fig. 3: den Befestigungsnagel von Figur 1 und Figur 2 im axialen Längsschnitt;
- Fig. 4: ein weiteres Ausführungsbeispiel eines Setzwerkzeuges nach der Erfindung mit Einsetzhilfe im axialen Längsschnitt;
- Fig. 5: die Einsetzhilfe des Setzwerkzeuges von Figur 4 in der Draufsicht;
- Fig. 6 - 6a): den Haltefuß der Einsetzhilfe von Figur 5 in vergrößerter Detaildarstellung, ebenfalls in der Draufsicht;
- Fig. 6b): einen Schnitt entlang der Linie VIb - VIb von Figur 6a), in Richtung der Pfeile gesehen;
- Fig. 7a): den Haltefuß der Einsetzhilfe von Figur 5 mit in die Bohrstellung vorgeschobener Bohrschablone; und
- Fig. 7b): einen Schnitt entlang der Linie VIIb - VIIb von Figur 7a, in Richtung der Pfeile gesehen.

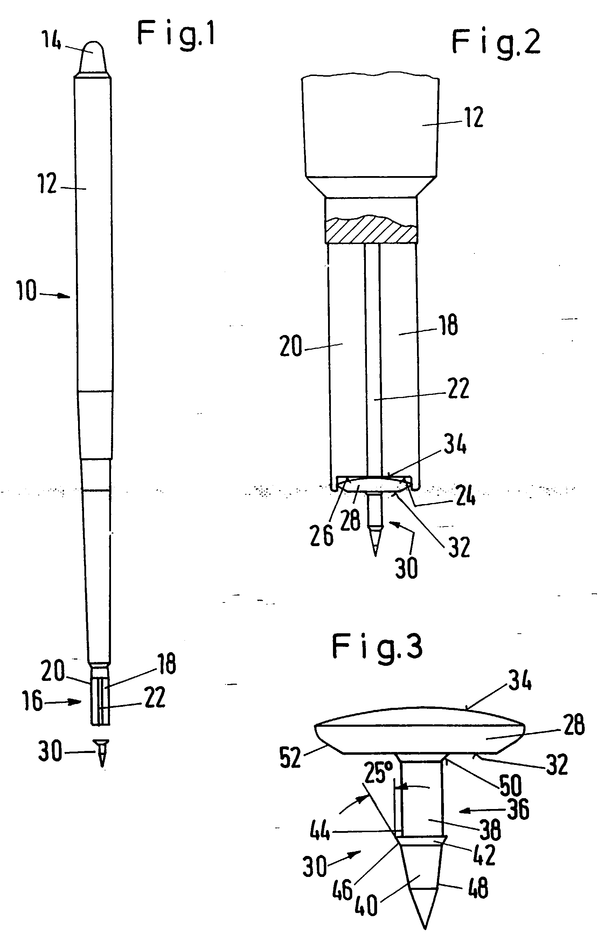

Wie Figur 1 erkennen läßt, weist das dort gezeigte Ausführungsbeispiel eines Setzwerkzeuges 10 nach der Erfindung ein langgestrecktes, "bleistiftartiges" Griffteil 12 auf, an dessen in der Zeichnung oben gezeigtem Ende ein Druck-Schlag-Kopf 14 zur Beaufschlagung z. B. mit einem Hammer vorgesehen ist. An dem dem Druck-Schlag-Kopf 14 entgegengesetzten Ende des Setzwerkzeuges 10 befindet sich eine Halteeinrichtung 16, die, wie die vergrößerte Darstellung von Figur 2 besser erkennen läßt, vier Greifbacken 18, 20 aufweist, welche durch eine Kreuzschlitzanordnung 22 voneinander getrennt sind und die von dem Griffteil 12 aus federnd vorspringen. An ihrem dem Griffteil 12 abgewandten Stirnende sind die Greifbacken 18, 20 mit Anlageschultern 24, 26 versehen.

Ein Nagelkopf 28 eines Befestigungsnagels 30, der weiter unter Bezugnahme auf Figur 3 noch näher erläutert wird, hat, in Axialrichtung gesehen, eine größere Höhe zwischen einer Preßfläche 32 desselben und dem "höchsten" Punkt einer Kopffläche 34, die größer ist als der Abstand der Anlageschultern 24, 26 vom Stirnende der Greifbacken 18, 20.

Wie Figur 3 erkennen läßt, weist der Befestigungsnagel 30 nach der Erfindung bei dem dort gezeigten Ausführungsbeispiel neben dem bereits erwähnten Nagelkopf 28 mit Kopffläche 34 und Preßfläche 32 einen Nagelschaft 36 auf, der aus einem im wesentlichen zylindrischen Halteteil 38 und einem in der Zeichnung unten gezeigten Spitzenteil 40, einstückig hiermit susgebildet, besteht, wobei zwischen dem Halteteil 38 und dem Spitzenteil 40 eine Ringschulter 42 vorgesehen ist. Die Ringschulter 42 geht mit einer im wesentlichen senkrecht zur Längsachse des Befestigungsnagels 30 verlaufenden Ringfläche 44 in die Mantelfläche des Halteteiles 38 über. An die Ringfläche 44 schließt in Richtung auf die Nagelspitze hin eine Konusfläche 46 an, deren Winkel mit der Längsachse des Befestigungsnagels bei dem gezeigten Ausführungsbeispiel 25° beträgt, wobei dieser Winkel größer ist als derjenige, den die Mantelfläche 48 des Spitzenteiles 40 mit der Längsachse des Befestigungsnagels bildet.

Die Mantelfläche des Halteteiles 38 des Befestigungsnagels 30 geht mit einer Anschrägung 50 in die Preßfläche 32 des Nagelkopfes 28 über. Von der Preßfläche 32 des Nagelkopfes 28 steigt zu dessen größtem Außenumfang eine Umfangsschrägung 52 an.

Der gesamte Nagel ist aus einer körperfreundlichen Titanlegierung hergestellt.

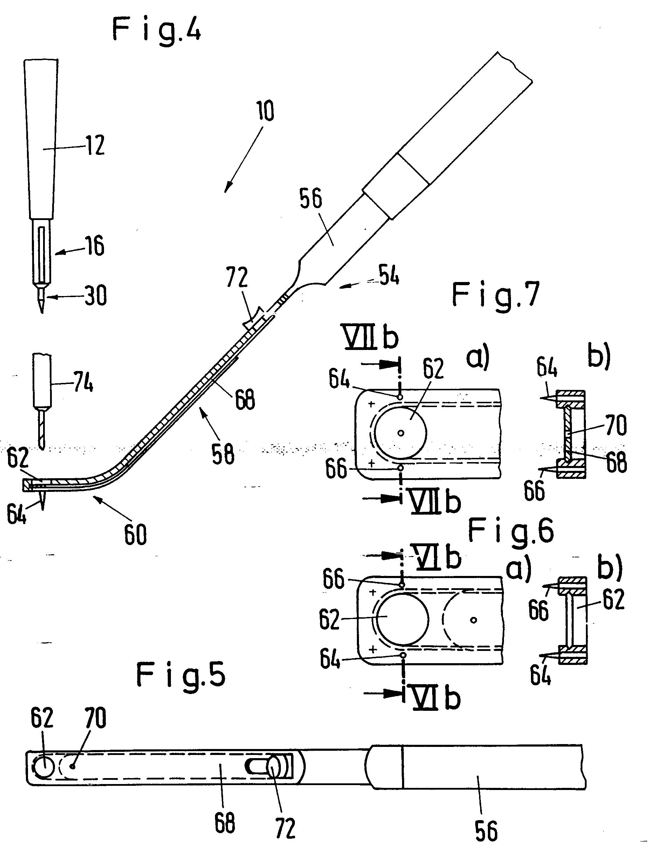

Bei dem in Figur 4 gezeigten Ausführungsbeispiel weist das Setzwerkzeug eine Einsetzhilfe 54 auf, die im wesentlichen aus einem Griffteil 56 und einem Fixierteil 58 besteht. Das Fixierteil 58 weist einen Haltefuß 60 auf, der mit einer Setzöffnung 62 versehen ist, an deren beiden Seiten diametral einander gegenüberliegend zwei Haltedorne 64, 66 (siehe hierzu auch Figur 6 und 7) angeordnet sind. Eine aus biegsamem Metallblech bestehende Bohrschablone 68 weist an ihrem dem Griffteil 56 abgewandten Ende eine Bohröffnung 70 und an ihrem dem Griffteil 56 zugewandten Ende ein Betätigungselement 72 auf.

Die Erfindung gemäß dem gezeigten und beschriebenen Ausführungsbeispiel wird die folgt angewendet:

Im einfachsten Fall, wenn also kein vorheriges Einbringen einer Setzbohrung in den körrereigenen festen Knochen notwendig ist, wird ein Befestigungsnagel 30 in der aus Figur 2 ersichtlichen Form in die Halteeinrichtung 16 des Setzwerkzeuges 10 eingesetzt. Die Greifbacken 18, 20 halten dabei den Nagelkopf 28 federnd, wobei die Kopffläche 34 an den Anlageschultern 24, 26 der Greifbacken 18, 20 anliegt. Infolge der Dimensionierung, die oben geschrieben wurde, liegt die Preßfläche 32 des Befestigungsnagels 30 dabei aus "Knochenrichtung" gesehen vor den Stirnenden der Greifbacken 18, 20, wie dies auch in Figur 2 ohne weiteres erkennbar ist. Das Griffteil 12 des Setzwerkzeuges 10 wird vom Operateur mit der Hand erfaßt, woraufhin der Befestigungsnagel 30 in den Knochen eingedrückt oder unter Zuhilfenahme eines den Druck-Schlag-Kopf 14 beaufschlagenden Hammers in den Knochen eingetrieben wird. Hierdurch wird die auf dem Knochen aufliegende Abdeckmembran fest zwischen der Preßfläche 32 des Nagelkopfes 28 und der daran anliegenden Knochenoberfläche eingeklemmt, wobei die Anschrägung 50 verhindert, daß sich in einem "toten Winkel" unerwünschte Schleimhautreste oder dergleichen ansammeln können. Durch das dichte Anlegen der nicht gezeigten Abdeckmembran an dem Knochen wird verhindert, daß in die mittels der Abdeckmembran abgedeckte Defektstelle des Knochens, die mit Knochenersatzmaterial ausgefüllt ist, weiches Bindegewebe, insbesondere Schleimhautgewebe, eindringen und einwachsen kann, wodurch ein knöchernes Durchwachsen der Knochenersatz- oder aufbaumasse gewährleistet wirkt. Die Anlageschulter 42, die widerhakenartig wird, gewährleistet dabei einen festen Sitz des Befestigungsnagels 30 im Knochen. Die Umfangsschrägung 52 ermöglicht es, den Befestigungsnagel 30 gewünschtenfalls durch Ansetzen einer Zange oder dergleichen wieder aus dem Knochen herauszuheben.

Im allgemeinen ist der körpereigene Knochen jedoch zu hart, als daß der Befestigungsnagel 30 ohne vorheriges Einbringen einer Setzbohrung in den Knochen eingesetzt werden könnte. Dann wird wie folgt vorgegangen: Mittels der in den Figuren 4 bis 7 gezeigten Setzhilfe 54 wird die Abdeckmembran an den Knochen angedrückt, wobei die Haltedorne 64, 66 die Membran punktuell lateral unverschiebbar am Knochen festlegen. Der Haltefuß 60 der Setzhilfe 54 liegt dabei flach an dem Knochen bzw. der diesen abdeckenden Abdeckmembran an. Bei vorgeschobener Bohrschablone 68, wobei also die Setzöffnung 62 bis auf die Bohröffnung 70 vollständig verschlossen ist (Figur 7), wird mittels eines Bohrers 74 durch die Bohröffnung 70 hindurch eine Setzbohrung in den Knochen eingebracht. Dann wird der Bohrer 74 herausgezogen und die Bohrschablone 68 zurückgezogen, ohne daß der Haltefuß 60 von dem mit der Abdeckmembran abgedeckten körpereigenen Knochen abgehoben würde. Alsdann wird durch die nunmehr freiliegende Setzöffnung 62 hindurch mittels des Setzwerkzeuges 10, dessen Griffteil 12 der Operateur mit seiner freien Hand hält, der Befestigungsnagel 30 in die Setzbohrung eingesetzt. Der Durchmesser der Setzbohrung ist dabei vorzugsweise so gewählt, daß er etwa dem Außendurchmesser des Halteteiles 38 des Befestigungsnagels 30 entspricht, jedoch kleiner ist als der Außendurchmesser der Ringschulter 42, so daß letztere auch in diesem Fall ihre widerhakenartige Haltefunktion ausüben kann.

Die in der vorstehenden Beschreibung, in den Ansprüchen sowie in der Zeichnung offenbarten Merkmale der Erfindung können sowohl einzeln als auch in beliebiger Kombination für die Verwirklichung der Erfindung in ihren verschiedenen Ausführungsformen wesentlich sein.

### BEZUGSZEICHENLISTE

- 10: Setzwerkzeug
- 12: Griffteil
- 14: Druck-Schlag-Kopf
- 16: Halteeinrichtung
- 18: Greifbacken
- 20: Greifbacken
- 22: Kreuzschlitzanordnung
- 24: Anlageschulter
- 26: Anlageschulter
- 28: Nagelkopf
- 30: Befestigungsnagel
- 32: Preßfläche
- 34: Kopffläche
- 36: Nagelschaft
- 38: Halteteil
- 40: Spitzenteil
- 42: Ringschulter
- 44: Ringfläche
- 46: Konusfläche
- 48: Mantelfläche
- 50: Anschrägung
- 52: Umfangsschrägung
- 54: Setzhilfe
- 56: Griffteil
- 58: Fixierteil
- 60: Haltefuß
- 62: Setzöffnung
- 64: Haltedorn
- 66: Haltedorn
- 68: Bohrschablone
- 70: Bohröffnung
- 72: Betätigungselement
- 74: Bohrer

## Patentansprüche

1. Setzwerkzeug (10) zum Einsetzen eines Befestigungsnagels umfassend einen Nagelkopf und einen daran anschließenden Nagelschaft, gekennzeichnet durch ein langgestrecktes bleistiftartiges Griffteil (12), an dessen einem Ende ein Druck-Schlag-Kopf (14) und an dessen anderem Ende eine mit mindestens zwei federnden Greifbacken (18,20) versehene Halteeinrichtung (16) für einen Befestigungsnagel (30) vorgesehen ist.

2. Setzwerkzeug nach Anspruch 1, dadurch gekennzeichnet, daß die Halteeinrichtung (16) vier durch eine Kreuzschlitzanordnung (22) getrennte Greifbacken (18, 20) aufweist.

3. Setzwerkzeug nach Anspruch 1 oder 2, dadurch gekennzeichnet, daß die Greifbacken einstückig mit dem daran anschließenden Bereich des Mantels des Griffteiles (12) ausgebildet sind.

4. Setzwerkzeug nach einem der Ansprüche 1 bis 3, dadurch gekennzeichnet, daß die Greifbacken (18, 20) jeweils eine Anlageschulter (24, 26) für die Kopffläche (34) des Befestigungsnagels (30) aufweisen, deren Abstand von dem dem Griffteil (12) abgewandten Stirnende der Halteeinrichtung (16) kleiner ist als die Höhe des Nagelkopfes (28).

5. Setzwerkzeug nach einem der Ansprüche 1 bis 4, gekennzeichnet durch eine von dem Griffteil (12) unabhängige Setzhilfe (54), mit einem Griffteil (56), einem daran anschließenden Fixierteil (58), welches einen mit einer Setzöffnung (62) versehenen, an den Knochen in Anlage bringbaren Haltefuß (60) aufweist, deren Durchmesser geringfügig größer ist als der Außendurchmesser der Halteeinrichtung (16), und einer in dem Fixierteil (58) längsverschiebbaren Bohrschablone (68) aus biegsamem Blech oder dergleichen, die an ihrem der Setzöffnung (62) benachbarten Ende eine Bohröffnung (70) aufweist und aus einer Bohrstellung, in der die Setzöffnung (62) bis auf die Bohröffnung (70) verschlossen ist, in eine Setzstellung zurückziehbar ist, in der die Setzöffnung (62) freiliegt.

6. Setzwerkzeug nach Anspruch 5, dadurch gekennzeichnet, daß der Haltefuß (60) an der an dem Knochen ansetzbaren Unterseite mindestens zwei spitze Haltedorne (64, 66) aufweist.

7. Setzwerkzeug nach Anspruch 6, dadurch gekennzeichnet, daß diametral gegenüberliegend zu beiden Seiten der Setzöffnung (62) je ein Haltedorn (64, 66) vorgesehen ist.

8. Setzwerkzeug nach einem der Ansprüche 5 bis 7, dadurch gekennzeichnet, daß die Bohrschablone (68) nahe dem Griffteil (56) mit einem von einem Operateur ohne Loslassen des Griffteiles (56) betätigbaren Betätigungselement (72) versehen ist.
